# EUROPÄISCHE PATENTANMELDUNG

(11) **EP 4 640 221 A2**
(43) Veröffentlichungstag der Anmeldung: **29.10.2025**
(21) Anmeldenummer: 25194036.7
(22) Anmeldetag: 10.03.2023
(51) Int. Cl.: A61K 31/4741

(54) **TIERFUTTER ODER TIERFUTTERZUSATZ MIT CANADIN UND/ODER CANADIN-DERIVAT**

(30) Priorität: 11.03.2022 EP 22161642
(62) Teilanmeldung aus: 23711004.4
(71) Anmelder: Phytobiotics Futterzusatzstoffe GmbH, 65343 Eltville (DE)
(72) Erfinder: ROTH, Hermann, 65346 Eltville (DE)
(74) Vertreter: Richardt Patentanwälte PartG mbB

(57) **Zusammenfassung**

Die Erfindung betrifft ein Tierfutter, Tränkwasser oder Tierfutterzusatz beinhaltend Canadin und/oder ein Canadin-Derivat.

## Beschreibung

### Technisches Gebiet

Die Erfindung bezieht sich auf einen Tierfutterzusatz zur Optimierung des Fleisch-zu-Fett-Anteils in Nutztieren.

### Stand der Technik

Lebensmittel tierischer Herkunft stellen einen wichtigen Bestandteil der menschlichen Ernährung dar. In den letzten 20 Jahren ist insbesondere Fleisch mit einem hohen Muskel-zu-Fett Verhältnis verstärkt nachgefragt worden, da dieses Fleisch als besonders gesund gilt. Es enthält weniger Fette, insbesondere das als gesundheitlich bedenklich geltende Cholesterin, dafür aber einen höheren Anteil an Eiweiß.

Am deutlichsten ist der Rückgang des Fettgehaltes bei Schweinefleisch. Enthielten 100 Gramm Schweinefilet 1991 noch knapp 9 Gramm Fett, sind es heute gerade noch 2 Gramm.

Ein hoher Anteil an Muskelfleisch und ein geringer Fettgehalt werden als Basis für verschiedene Qualitätsmaße von Fleisch verwendet. Beispielsweise müssen sogenannte "Metzgerschweine" einen Muskelfleischanteil von mindestens 60% und ein Speckmaß von maximal 12 bis 14 mm aufweisen. Maximal 16 bis 20 mm Speck sind je nach Fleischmaß erlaubt, um noch in die Handelsklasse E (55 % und mehr) zu gelangen, während 22 bis 26 mm Speck je nach Fleischmaß die Grenze von der Handelsklasse U (50 bis 55%) zur Klasse R (45 bis 50%) darstellen. Das Speckmaß hat dabei einen sehr hohen Einfluss auf die Klassifizierung des Fleisches in die verschiedenen Handels- bzw. Qualtätsklassen, denn das Speckmaß wird in der derzeit gültigen Formel 4,5-mal so stark gewichtet wie das Fleischmaß. Wenn das Speckmaß um 2 mm zunimmt, muss also das Fleischmaß um 9 mm steigen, um eine Verschlechterung bei der Fleischklassifizierung zu vermeiden.

Die bisher verwendeten Methoden zur Erlangung eines optimalen, also hohen, Fleisch-zu-Fett-Verhältnisses, sind jedoch mit verschiedenen Nachteilen verbunden. Oft bewirken sie einen erheblichen Verlust an verwertbarem tierischen Material und/oder gesteigerte Produktionskosten.

So ist es beispielsweise möglich, die Fleischzuschnitte so zu gestalten, dass von vornehrein das angrenzende Fett weggeschnitten wird. Das weggeschnittene Fett hat jedoch einen deutlich geringeren Marktwert, sodass die Kosten für das zu seinem Aufbau eingesetzte Futter als verloren oder zumindest subobtimal eingesetzt anzusehen sind. Fett, welches sich innerhalb des Muskelgewebes befindet, lässt sich auf diese Weise gar nicht entfernen.

Eine weitere Möglichkeit der Erzeugung von Fleisch mit dem gewünschten hohen Flesich-zu-Fett Verhältnis besteht darin, Zuchtrassen mit einem dahingehend optimierten Fleisch zu verwenden. Dies kann aber mit verschiedenen Nachteilen verbunden sein: bestehende Ställe oder Haltungskonzepte können für die besagten Zuchtrassen ungeeignet sein, die besagten Rassen können teuer oder krankheitsanfällig und/oder für das vorherrschende Klima nicht geeignet sein. Eine Anpassung der Haltungsvorrichtungen die Bedürfnisse dieser speziellen Rassen kann mit erheblichen Kosten verbunden sein, und sie verringert die Flexibilität des Landwirts, kurzfristig geänderte Kundenwünsche bedienen zu können.

Eine weitere Möglichkeit besteht darin, sehr junge Tiere, die noch kaum Fett angesetzt haben, zu schlachten. Dies kann jedoch dazu führen, dass die Tiere vor dem Alter geschlachtet werden, an welchem das optimale Wachstum (gemessen an der pro Futtermenge erzeugten Fleischmenge) erreicht ist. Die Produktionseffizienz sinkt, die Kosten steigen.

Die Bereitstellung von zusätzlichem Auslauf kann den Muskelfleischanteil erhöhen, doch auch die Produktionskosten steigen dadurch erheblich.

Ein weiterer Ansatz besteht darin, die Fütterung so anzupassen, dass dadurch ein im Rahmen des genetisch bei der jeweils verwendeten Rasse Möglichen ein hohes Fleisch-zu-Fett-Verhältnis und/oder ein möglichst geringer Fettanteil erzielt wird. Beispielsweise werden Futtermittel verwendet, die einen besonders hohen Anteil an Eiweiß und Aminosäuren, insbesonere Lysin, aufweisen. Zudem kann während der Endmast der Energiegehalt des Futters reduziert werden, um eine Verfettung der Tiere zu verhindern. Allerdings sind auch diesem Ansatz Grenzen gesetzt, z.B. durch den Preis der Futterkomponenten bzw. Zusatzstoffe und durch die physiologischen Bedürfnisse der jeweiligen Tierart an das Futter.

### Technisches Problem und grundlegende Lösungen

Der Erfindung liegt demgemäß die Aufgabe zugrunde, ein alternatives bzw. verbesserte Tierfutter bzw. Tränkwasser bzw. einen entsprechenden Tierfutterzusatz bereitzustellen.

Die der Erfindung zugrunde liegenden Aufgaben werden jeweils mit den Merkmalen der unabhängigen Patentansprüche gelöst. Ausführungsformen der Erfindung sind in den abhängigen Ansprüchen angegeben. Die im Folgenden aufgeführten Ausführungsformen und Beispiele sind frei miteinander kombinierbar, sofern sie sich nicht gegenseitig ausschließen.

In einem Aspekt betrifft die Erfindung ein Tierfutter, Tränkwasser oder Tierfutterzusatz beinhaltend Canadin und/oder beinhaltend ein Canadin-Derivat.

In mehreren Versuchen konnte beobachtet werden, dass die Verabreichung von Canadin-haltigem bzw. Canadinderivat-haltigem Futter an Nutztiere wie z.B. Schweine sich ausgesprochen positiv auf das Muskel-zu-Fett-Verhältnis und den Fettgehalt auswirkt: Der Fettanteil nimmt deutlich ab, der Anteil an Muskeln steigt. Somit wird dem Tierhalter ein weiteres Mittel an die Hand gegeben, um das Muskel-zu-Fett-Verhältnis zusätzlich oder alternativ zu den im Stand der Technik bekannten Verfahren zu verändern, bzw. den Fettanteil zu reduzieren, um die Qualität des Fleisches zu erhöhen. Beispielsweise könnte ein Tierhalter durch Zugabe von Canadin zum Futter während der Endmast es ermöglichen, dass die Tiere weiterhin ein Futter mit einem üblicherweise verwendeten Energiegehalt bekommen und dadurch schneller das Schlachtgewicht erreichen als Tiere, die während der Endmast energiereduziertes Futter erhalten haben, ohne dass dadurch der Gehalt an Magerfleisch im Schlachttier abnimmt. Alternativ könnte der Tierhalter durch Zugabe von Canadin bzw. einem Canadin-Derivat zu einem energiereduzierten Futter während der Endmast erreichen, dass die Tiere zum Ende der Mast einen besonders hohen Anteil an Magerfleisch, einen besonders geringen Fettanteil und ein besonders hohes Muskel-zu-Fett Verhältnis haben. Dieses Verhältnis wäre höher als durch die Verabreichung von energiereduziertem Futter alleine erzielbar wäre.

Gegenüber zumindest einigen der im Stand der Technik bekannten Verfahren hat die Verabreichung von Canadin- bzw. Canadin-Derivat-haltigem Futter bzw. Tränkwasser insbesondere auch den Vorteil der größeren Flexibilität: die Anmelderin hat festgestellt, dass Canadin bzw. Derivate des Canadins bei einer großen Zahl von Tierarten, darunter z.B. Rinder, Schweine, Hühner, und andere, das Muskel-zu-Fett-Verhältnis erhöht. Um ein gewünschtes, hohes Muskel-zu-Fett Verhältnis zu erreichen, muss also nicht zwingend eine bestimmte Tierrasse für die Mast gewählt und die Haltungsbedingungen an diese angepasst werden. Vielmehr kann der Tierhalter je nach voraussichtlichem Wunsch der Abnehmer in einigen Monaten den Magerfleischanteil und den Fettanteil der Tiere durch die Zugabe von Canadin flexibel kontrollieren und anpassen.

Außerdem kann der Tierhalter flexibel auf verschiedene unvorhersehbare Situationen während der Mast reagieren. Beispielsweise kann es vorkommen, dass gegen Ende der Mast ein erheblicher Anteil der Tiere eines Tierstalls mit einem Krankheitserreger infiziert wird, der die befallenen Tiere schwächt. In so einer Situation ist es nicht angezeigt, energiereduziertes Futter zu verabreden, da die befallenen Tiere Energie zur Bekämpfung des Erregers benötigen. Allerdings erfolgt die Futterabgabe in großen Ställen zentral. Wird den nicht vom Erreger befallenen Tieren ein hochkalorisches Futter verabreicht, besteht die Gefahr, dass die gesunden Tiere verfetten, also einen unerwünscht hohen Fettanteil aufweisen. Der Tierhalter muss in so einer Situation also abwägen zwischen dem Risiko, durch Verabreichung energiereduzierten Futters zur Endmast den Gesundheitszustand der bereits befallenen Tiere weiter zu verschlechtern und das Risiko zu erhöhen, dass der Erreger auf andere Tiere überspringt, und dem Risiko, durch Verabreichung eines hochenergetischen Futters zwar den befallenen Tieren zu helfen, aber die Fleschqualität der nicht-befallenen Tiere zu beeinträchtigen. In solchen Situationen kann die Verabreichung von Futtermitteln oder von Tränkwasser mit Canadin bzw. einem CanadinDerivat, optional in Kombination mit Medikamenten zur Behandlung einer Tierkrankheit (wie z.B. Kokzidiose, Durchfallerkrankungen, Newcastle Krankheit, etc.) dafür sorgen, dass eine hohe Fleischqualität auch in Situationen gewährleistet ist, in welchen wegen des Vorliegens oder des Verdachts auf Vorliegens einer Krankheit ein energiereiches Futter verabreicht werden muss. Auch in anderen Situationen, in welchen zu fürchten ist, dass die Fleischqualität im Hinblick auf den Fett- und Muskelfleischanteil bei den Schlachttieren zu niedrig sein wird, z.B. wegen des Geschlechts, der Rasse der Tiere, dem Alter, dem Gesundheitszustands oder wegen des verwendeten Futters, kann Canadin-haltiges Futter bzw. Tränkwasser dafür sorgen, dass eine deutliche Verschiebung des Muskel-zu-Fett-Verhältnisses zugungsten des Muskel-Anteils und zulaten des Fettanteils stattfindet.

Canadin, auch als Tetrahydroberberin bzw. unter der CAS Nummer 522-97-4 bekannt, ist ein Benzylisochinolin-Alkaloid (BIA) aus der strukturellen Untergruppe der Protoberberin-Alkaloide. Canadin enthält - wie auch die anderen Protoberberin-Alkaloide - eine Benzylisochinolin-Struktureinheit. Canadin gehört somit zu der Gruppe der Alkaloide.

Unter einem Canadin-Derivat wird hier physiologisch verträgliche Substanz verstanden, die sich von Canadin als Stammverbindung ableitet, also die Grundstruktur des Canadins enthält, zusätzlich aber auch ein oder mehrere funktionale Gruppen. Insbesondere kann es sich bei dem Canadin-Derivat um eine methylierte Form des Canadins mit identischer oder Ähnlicher Wirkung auf den Fleisch- und Fettanteil eines Tieres wie Canadin handeln. Beispielsweise kann es sich bei dem Canadin-Derivat um einfach oder mehrfach methylierte Varianten des Canadins handeln. Insbesondere kann es sich bei dem Canadin-Derivat um einfach methyliertes Canadin, insbesondere das N-Methylcanadin handeln. Beispielswiese kann es sich bei dem N-Methylcanadin um das (S)-N-Methylcanadin mit der CHEBI Nummer 16512 handeln.

Nach einer Ausführungsform handelt es sich um Tierfutter oder Tränkwasser mit einer Konzentration von zumindest 0,16 µg Canadin und/oder Canadin-Derivat pro kg Tierfutter bzw. Tränkwasser, vorzugsweise mindestens 1,6 µg Canadin und/oder Canadin-Derivat pro kg Tierfutter bzw. Tränkwasser, z.B. von 1 µg bis 50 µg Canadin und/oder Canadin-Derivat pro kg Tierfutter bzw. Tränkwasser.

Nach einer anderen Ausführungsform handelt es sich um einen Tierfutterzusatz, der bei bestimmungsgemäßer Beimengung zu einem Tierfutter oder zu einem Tränkwasser ein Tierfutter bzw. Tränkwasser mit einer Konzentration von zumindest 0,16 µg Canadin und/oder Canadin-Derivat pro kg Tierfutter bzw. Tränkwasser, vorzugsweise mindestens 1,6 µg Canadin und/oder Canadin-Derivat pro kg Tierfutter bzw. Tränkwasser, z.B. von 1 µg bis 50 µg Canadin und/oder Canadin-Derivat pro kg Tierfutter bzw. Tränkwasser, ergibt.

Beispielsweise kann es sich bei dem Tierfutterzusatz um ein Pulver oder Granulat handeln, welches Canadin und/oder ein Canadin-Derivat, optional ein oder mehrere weitere Alkaloide sowie typischerweise noch andere Stoffe, z.B. Füllstoffe, Mineralien, Vitamine und/oder Aromastoffe enthält. Der Tierfutterzusatz kann auch Pflanzenmaterial, z.B. zerriebene Pflanzenteile wie z.B. Blätter oder Stengel und/oder Pflanzenextrakte enthalten, in welchen typischerweise die Alkaloide enthalten sind. Die Füllstoffe erleichtern die Dosierung und die homogene Beimengung des Tierfutterzusatzes zu einer deutlich größeren Menge Tierfutter oder Wasser, sodass die Endkonzentration der Wirkstoffe auf die Bedürfnisse der Tiere und der jeweiligen Anwendungssituation angepasst werden kann. Der Tierfutterzusatz kann auch in flüssiger Form vorliegen. Letzteres ist besonders vorteilhaft, wenn der Zusatz mit Wasser vermengt werden soll um ein Canadin-haltiges Tränkwasser einer bestimmten Konzentration zur Verfügung zu stellen. Beispielsweise kann das Canadin und/oder Canadin-Derivat durch Absenkung des pH Werts des flüssigen Futterzusatzes (z.B. mittels organischer Säuren wie z.B. Zitronensäure) in Lösung gehalten werden.

Nach einer Ausführungsform beinhalten das Tierfutter, der Tierfutterzusatz oder das Tränkwasser Berberrubin.

Dieser Ausführungsform liegt die überraschende Beobachtung zu Grunde, dass die Verwendung einer Kombination von Canadin und/oder einem Canadin-Derivat in Kombination mit Berberrubin besonders vorteilhaft sein kann, da Berberrubin die Wirkung des Canadins bzw. Canadin-Derivats zu verstärken scheint. Durch die Verabreichung einer Kombination beider Substanzen konnte eine deutlich stärkere Verbesserung des Muskelfleisch-zu-Fett Verhältnisses erzielt werden als dies bei einer alleinigen Verabreichung von Canadin beobachtbar war. Auch die Lagerfähigkeit des Fleisches verbesserte sich.

Beispielsweise kann das Berberrubin in Form von Pflanzenmaterial in dem Tierfutter, Tierfutterzusatz oder Tränkwasser enthalten sein. Beispielsweise kann es sich bei dem Pflanzenmaterial um Material der Pflanzen Thalictrum aquilegifolium, Berberis heteropoda, um Material aus Zweigen von Berberis actinacantha oder aus den Stämmen von Berberis darwinii oder Berberis valdiviana handeln. Auch aus Berberis vulgaris, Thalictrum polygamum, Fibraurea chloroleuca (Berberidaceae, Ranunculaceae, Menispermaceae), Macleya cordata sowie weiteren Pflanzen kann Berberrubin in hinreichender Konzentration enthalten sein oder in Form eines Extrakts gewonnen werden. Beispielsweise kann es sich bei dem Extrakt um einen wässrig-ethanolischen Auszug aus Pflanzenmaterial handeln.

Nach einer Ausführungsform liegt das Verhältnis der Canadinkomponente (Canadin und/oder Canadinderivat) zu Berberrubin im Tierfutter, Tierfutterzusatz und/oder im Tränkwasser im Bereich von 1:5 bis 5:1, z.B. im Bereich von 1:1,2 bis 1,2:1.

Nach Ausführungsformen der Erfindung enthält das Tierfutter, der Tierfutterzusatz oder Tränkwasser getrocknete Teile der Pflanze Maclaeaya cordata, z.B. Blätter und/oder Wurzeln, Blüten, Kapseln und/oder Teile des Stamms. Insbesondere die Blüte und die Kapsel aber auch die Wurzel enthält Canadin und insbesondere auch einen hohen Anteil an N-methyl-Canadin. Durch Kombination von Blättern mit verschiedenen Anteilen von Blüten, Kapseln und/oder Wurzeln können so je nach Anwendungszweck und Wirkstoffgehalt der Pflanze die gewünschte Konzentration an Canadin und N-methyl-Canadin leicht eingestellt werden. Die getrockneten Teile werden vorzugsweise zuvor zu einem Pulver vermahlen um die Dosierung und Mischung zu vereinfachen.

Nach einer Ausführungsform beinhaltet das Tierfutter oder Tränkwasser zumindest 1 µg Berberrubin pro kg Tierfutter bzw. Tränkwasser, insbesondere zumindest 10 µg Berberrubin pro kg Tierfutter bzw. Tränkwasser. Die Berberrubinkonzentration kann aber auch höher liegen, z.B. im Bereich von 1-400 µg Berberrubin pro kg Tierfutter bzw. Tränkwasser.

Nach einer weiteren Ausführungsform weist der Tierfutterzusatz eine solche Berberrubinkonzentration auf, dass sich bei bestimmungsgemäßer Beimengung zu einem Tierfutter oder zu einem Tränkwasser ein Tierfutter oder Tränkwasser mit zumindest 1 µg Berberrubin pro kg Tierfutter bzw. Tränkwasser, insbesondere zumindest 10 µg Berberrubin pro kg Tierfutter bzw. Tränkwasser, ergibt. Auch Konzentrationen von 1-400µg Berberrubin pro kg Tierfutter oder Tränkwasser sind möglich.

Nach manchen Ausführungsformen kann das Tierfutter oder das Tränkwasser bzw. der für deren Erzeugung verwendete Tierfutterzusatz weitere Alkaloide wie z.B. Sanguinarin und/oder Chelerythrin enthalten, die verschiedene positive Effekte haben können, z.B. eine Verbesserung der Futterverwertung.

Nach einer Ausführungsform ist das Tierfutter, der Tierfutterzusatz oder das Tränkwasser bestimmt für die Verabreichung an Nutztiere, Haustiere oder Hobbytiere. Bei den Tieren kann es sich insbesondere um Geflügel, Schweine, Rinder, Schafe, Ziegen, Pferde, und/oder Kaninchen handeln.

Nach manchen Ausführungsformen handelt es sich bei dem in dem Tierfutter, Tränkwasser oder Tierfutterzusatz enthaltenen Canadin um synthetisch oder biotechnologisch, insbesondere mikrobiotechnologisch erzeugtes Canadin.

Beispielsweise kann Canadin in mehreren Schritten aus einem disubstituierten Phenylethylamin und einem disubstituierten Benzaldehyden gewonnen werden, wie dies z.B. in Li W et al., "Total Synthesis of (-)-Canadine, (-)-Rotundine, (-)-Sinactine, and (-)-Xylopinine Using a Last-Step Enantioselective Ir-Catalyzed Hydrogenation", Journal Organical Chemistry 2021 Jun 18;86(12):8143-8153. doi: 10.1021/acs.joc.1c00602. Epub 2021 Jun 2. PMID: 34076443 beschrieben ist. Die Verwendung synthetischen Canadins hat den Vorteil, dass eine exakte Dosierung erleichtert wird. Bei der Verwendung von Pflanzenmaterial können ggf. zusätzliche Schritte zur Aufkonzentration, Reinigung und/oder zur Bestimmung der Canadin-Konzentration im verwendeten Pflanzenmaterial erforderlich sein.

Nach anderen Ausführungsformen hadelt es sich um Canadin, das als Bestandteil von Pflanzenmaterial, insbesondere eines Pflanzenextrakts, in dem Tierfutter, Tränkwasser oder Tierfutterzusatz vorliegt.

Beispielsweise kann das Tierfutter, der Tierfutterzusatz oder das Tränkwasser Pflanzenmaterial der kanadischen Orangenwurzel oder einer Pflanze der Familie der Papaveraceae, wie z. B. Corydalis yanhusuo und Corydalis turtschaninovii, enthalten. Gemäß eines anderen Beispiels kann das Pflanzenmaterial ganz oder teilweise vom weißen Federmohn (Macleaya cordata) kommen, insbesondere vom oberirdischen Teil, also von Stängeln und/oder Blättern.

Gemäß Ausführungsformen enthält das Tierfutter oder Tränkwasser mindestens 10 mg Macleaya cordata Blätter je kg Tierfutter oder Tränkwasser, insbesondere mindestens 100 mg Macleaya cordata Blätter je kg Tierfutter oder Tränkwasser. Vorzugsweise ist das Tierfutter oder Tränkwasser frei von anderen Pflanzenorganen von Macleaya cordata (wie etwa Wurzeln, Stengeln, Blüten, etc.) oder enthält diese anderen Pflanzenorgane in einer Menge von weniger als 20%, insbesondere weniger als 10%, z.B. weniger als 5% oder weniger als 1% des Mengenanteils der Macleaya cordata Blätter pro kg Tierfutter oder Tränkwasser. Die Zusammensetzung des Tierfutterzusatzes und die Menge an Macleaya cordata Blättern im Zusatz ist so gewählt, dass, bei bestimmungsgemäßer Dosierung, ein entsprechendes Tierfutter oder Tränkwasser erhalten wird. Dies kann vorteilhaft sein, weil M. cordata Blätter eine vergleichsweise hohe Konzentration insbesondere an N-methyl-Canadin enthalten und die Blätter einen Großteil der Biomasse von M. cordata Pflanzen ausmachen und leicht zu verarbeiten (z.B. pulverisieren) und homogen verteilbar sind.

Nach manchen Ausführungsformen handelt es sich bei dem in dem Tierfutter, Tränkwasser oder Tierfutterzusatz enthaltenen Canadinderivat um ein synthetisch, biotechnologisch und/oder mikrobiotechnologisch erzeugtes Canadinderivat. Beispielsweise ist ein Herstellungsverfahren von N-methyl-Canadin in der europäischen Patentanmeldung EP3221461A1 beschrieben.

Gemäß Ausführungsformen der Erfindung enthält das Tierfutter, Tränkwasser oder der Tierfutterzusatz eine Mischung aus Canadin und dem Canadin-Derivat.

Gemäß Ausführungsformen der Erfindung enthält das Tierfutter, Tränkwasser oder der Tierfutterzusatz eine Mischung aus synthetischem und/oder biotechnologisch erzeugtem Canadin und/oder Canadin pflanzlichen Ursprungs. Zusätzlich oder alternativ dazu kann das Tierfutter, Tränkwasser oder der Tierfutterzusatz eine Mischung aus dem synthetischem und/oder biotechnologisch erzeugtem Canadin-Derivat und/oder dem Canadin-Derivat pflanzlichen Ursprungs sein. Beispielsweise kann mittels einer Mischung aus synthetischen und pflanzenbasierten Wirkstoff gezielt eine gewünschte Wirkstoffkonzentration eingestellt werden, sodass natürliche Schwankungen im Wirkstoffgehalt von Pflanzen kompensiert werden können. Es ist jedoch auch möglich, z.B. durch geeignete Wahl des Anteils verschiedener Pflanzenorgane (Blätter, Stengel, Wurzeln, Blüten etc) eine gewünschte Wirkstoffkonzentration auf rein pflanzlicher Basis zu erhalten.

Nach manchen Ausführungsformen liegt das Canadin, das Canadin-Derivat und/oder das Berberrubin in dem Tierfutter, Tränkwasser oder Tierfutterzusatz als pysiologisch verträgliches Salz, insbesondere als Chlorid oder Sulfat, vor.

Die Verabreichung in Form von Salzen kann den Vorteil haben, dass das Alkaloid stabiliisert wird. Es wird davon ausgegangen, dass dies daran liegt, dass das Salz-Anion die Anlagerung von Hydroxyl-Ionen, die das Alkaloid inaktivieren können, verhindert.

Nach manchen Ausführungsformen handelt es sich bei dem Tierfutter, Tränkwasser oder Tierfutterzusatz um ein solches zur Verringerung des Fettanteils im Tierkörper und/oder Schlachtkörper.

Nach manchen Ausführungsformen handelt es sich bei dem Tierfutter, Tränkwasser oder Tierfutterzusatz um ein solches zur Erhöhung des Muskel-zu-Fett-Verhältnisses im Fleisch von Tieren. Nach manchen Ausführungsformen handelt es sich bei dem Tierfutter, Tränkwasser oder Tierfutterzusatz um ein solches zur Verwendung als Medikament, z.B. um ein Tierfutter, Tränkwasser oder Tierfutterzusatz zur Behandlung von Fettleibigkeit Muskelschwäche oder krankhaft erhöhtem oxidativen Stress.

Nach manchen Ausführungsformen handelt es sich bei dem Tierfutter, Tränkwasser oder Tierfutterzusatz um ein solches zur Erhöhung der Lagerfähigkeit des Fleisches geschlachteter Tiere.

Nach manchen Ausführungsformen handelt es sich bei dem Tierfutter, Tränkwasser oder Tierfutterzusatz um ein solches zur vorbeugenden oder akuten Behandlung von oxidativem Steress im Gewebe von Tieren, insbesondere während der Mast.

In einem weiteren Aspekt betrifft die Erfindung ein Tierfutter, Tränkwasser oder Tierfutterzusatz beinhaltend Berberrubin. Gemäß manchen Ausführungsformen beinhaltet das Tierfutter zusätzlich Canadin und/oder ein Canadin-Derivat. Die Dosierung, Konzentrationsangaben, Verwendungen und sonstige Eigenschaften, die hier für das Canadin-haltige Tierfutter, Tränkwasser oder Tierfutterzusatz beschrieben wurden, treffen gemäß Ausführungsformen auch auf das Tierfutter, Tränkwasser oder Tierfutterzusatz zu, welches Berberrubin (ohne Canadin und ohne Canadin-Derivat oder ggf. optional ergänzt durch Canadin und/oder das Canadin-Derivat) enthält.

In einem weiteren Aspekt betrifft die Erfindung eine Verwendung eines Tierfutters, Tränkwassers oder Tierfutterzusatzes nach einem der hier beschriebenen Ausführungsformen zur Verringerung des Fettanteils im Tierkörper und/oder im Schlachtkörper der Tiere.

In einem weiteren Aspekt betrifft die Erfindung eine Verwendung eines Tierfutters, Tränkwassers oder Tierfutterzusatzes nach einem der hier beschriebenen Ausführungsformen zur Erhöhung des Muskel-zu-Fett-Verhältnisses im Fleisch von Tieren und/oder im Schlachtkörper der Tiere.

In einem weiteren Aspekt betrifft die Erfindung eine Verwendung eines Tierfutters, Tränkwassers oder Tierfutterzusatzes nach einem der hier beschriebenen Ausführungsformen Verringerung des Fettanteils im Fleisch von Tieren und/oder im Schlachtkörper der Tiere.

In einem weiteren Aspekt betrifft die Erfindung eine Verwendung eines Tierfutters, Tränkwassers oder Tierfutterzusatzes nach einem der hier beschriebenen Ausführungsformen zur Erhöhung der Lagerfähigkeit des Fleisches geschlachteter Tiere.

In einem weiteren Aspekt betrifft die Erfindung eine Verwendung eines Tierfutters, Tränkwassers oder Tierfutterzusatzes nach einem der hier beschriebenen Ausführungsformen zur vorbeugenden oder akuten Behandlung von oxidativem Steress im Gewebe von Tieren, insbesondere während der Mast. Insbesondere kann das besagte Tierfutter, Tränkwasser oder Tierfutterzusatz zur vorbeugenden oder akuten Behandlung von pathologischem, also medizinisch behandlungsbedürftigem, oxidativem Steress im Gewebe von Tieren, verwendet werden.

Als oxidativen Stress bezeichnet man einen Zustand des Stoffwechsels eines Organismus, bei dem ein Übermaß reaktiver Sauerstoffverbindungen (ROS - reactive oxygen species) zu einer Schädigung der betroffenen Zellen führt. Diese reaktiven Sauerstoffverbindungen entstehen im Rahmen von Stoffwechselvorgängen wie etwa der Atmungskette. Beispiele für derartige Verbindungen sind das Superoxid-Anionenradikal O2^{·-}, Wasserstoffperoxid (H₂O₂) und das Hydroxylradikal ·OH. Normalerweise ist eine Zelle in der Lage, eine gewisse Menge reaktiver Sauerstoffverbindungen durch Neutralisation unschädlich zu machen. Dazu werden reduzierende Stoffe produziert und bevorratet. Oxidativer Stress ist dementsprechend ein derartiges Ungleichgewicht zwischen oxidierenden und reduzierenden Stoffen, dass diese normale Reparatur- und Entgiftungsfunktion der Zelle überfordert ist und folglich alle zellulären und extrazellulären Makromoleküle geschädigt werden können.

Zu den Folgen eines hochgradigen oxidativen Stresses gehören die Lipidperoxidation, die Proteinoxidation und die Schädigung der DNA. Diese drei Vorgänge gelten als mitursächlich für den Alterungsprozess und einen verschlechterten gesundheitlichen Zustand. Manche Formen des hochgradigen oxidativen Stresses sind als krankhafte, veterinärmedizinisch behandlungsbedürftige physiologische Zustände anzusehen, da sie die Wachstums- und Leistungsfähigkeit von Tieren in ähnlich erheblicher Weise einschränken wie dies bei vielen bekannten Tierkrankheiten wie z.B. Vogelgrippe, Kälberdurchfall, oder Kokzidiose der Falll ist.

Hochgradiger, insbesondere pathologischer oxidativer Stress entsteht, wenn der Zellstoffwechsel erheblich aus seinem Gleichgewicht gebracht wird. Dies kann z.B. durch eine vitalstoffarme Ernährung, schlechte Haltungsbedingungen, Infektionen, Entzündungen und sonstige gesundheitliche Probleme verursacht werden.

In manchen Ausführungsformen wird das Tierfutter, Tränkwasser oder der Tierfutterzusatz zur Verbesserung verschiedener Fleischparameter wie Fettgehalt, Verhältnis von Fett- und Muskelfleisch, Lagerfähigkeit etc. verwendet. In manchen Ausführungsformen wird das Tierfutter, Tränkwasser oder der Tierfutterzusatz als Medikament verwendet, z.B. zur Behandlung von Fettleibigkeit, oder von Muskelschwäche oder von einem krankhaft-schlechtem Allgemeinzustand aufgrund eines hohen Niveaus an oxidativem Stress im Gewebe. Die besagten Probleme können Alterserscheinungen sein oder durch schlechtes oder wenig ausgewogenes Futter hervorgerufen werden. Medizinische Verwendungen sind insbesdonere bei Haus- und Hobbytieren vorteilhaft, können jedoch auch bei der Nutztierhaltung erfolgreich eingesetzt werden, z.B. bei der Mast von Schweinen, Rindern und Geflügel.

In einem weiteren Aspekt betrifft die Erfindung ein Behältnis, welches den Tierfutterzusatz beinhaltet und welches zusätzlich einen Informationsträger, z.B. einen Aufdruck oder Aufkleber umfasst oder zusammen mit dem Informationsträger (z.B. Beipackzettel) bereitgestellt wird. Es ist auch möglich, dass der Informationsträger einen Verweis (URL, z.B. eine in einem QR-Code codierte URL) auf einen Dosierungshintweis enthält, der in elektronischer Form, z.B. als eine Webseite, zur Verfügung gestellt wird. Auf dem Informationsträger (und/oder auf dem referenzierten eletkronischen Informationsträger) ist eine Dosierung spezifiziert, also eine Spezifizierung derjenigen Menge an Tierfutterzusatz, die bei bestimmungsgemäßer Verwendung einer bestimmten Menge an Tierfutter oder Tränkwasser zugegeben werden muss, um ein Tierfutter oder Tränkwasser mit einer Konzentration an Canadin und/oder Canadin-Derivat und/oder Berberrubin zu erhalten, wie sie hier für Ausführungsformen der Erfindung beschrieben wurden. Bei dem Behältnis kann es sich z.B. um einen Eimer, eine Dose, einen Beutel, einen Sack oder auch deutlich kleinere oder größere Behältnisse handeln, je nachdem, ob die Menge für eine medizinische Verwendung zur Behandlung einzelner Haustiere oder für eine reguläre Futterbeimengung durch größere Mastbetriebe gedacht ist.

In einem weiteren Aspekt betrifft die Erfindung eine Verwendung eines Tierfutters, Tränkwassers oder Tierfutterzusatzes nach einem der hier beschriebenen Ausführungsformen für medizinische Zwecke bei Tieren. Beispielsweise kann das Tierfutter, Tränkwasser oder Tierfutterzusatz zur Behandlung von medizinisch behandlungsbedürftiger Adipositas bei Haustieren, krankhaft-hohen Konzentrationen an freien Sauerstoffradikalen (oxidativer Stress) im Gewebe oder krankhaft geringer Ausbildung von Muskeln oder Muskelprotein verwendet werden.

In einem weiteren Aspekt betrifft die Erfindung eine Verwendung eines Tierfutterzusatzes nach einer der hier beschriebenen Ausführungsfomen zur Herstellung eines Tierfutters oder Tränkwassers für die vorbeugende oder akute Behandlung von oxidativem Steress im Gewebe von Tieren.

In einem weiteren Aspekt betrifft die Erfindung eine Verwendung eines Tierfutterzusatzes nach einer der hier beschriebenen Ausführungsfomen zur Herstellung eines Tierfutters oder Tränkwassers für die Verringerung des Fettanteils im Tierkörper.

In einem weiteren Aspekt betrifft die Erfindung eine Verwendung eines Tierfutterzusatzes nach einer der hier beschriebenen Ausführungsfomen zur Herstellung eines Tierfutters oder Tränkwassers für die Erhöhung des Muskel-zu-Fett-Verhältnisses und/oder zur Reduktion des Fettanteils im Fleisch von Tieren.

Nach Ausführungsformen ist die Verwendung des Tierfutterzusatzes, Tierfutters oder Tränkwassers dadurch gekennzeichnet, dass bei bestimmugsgemäßer Dosierung des Tierfutterzusatzes, des Tierfutters und/oder des Tränkwassers pro Tag mindestens 0,016 µg Canadin und/oder Canadin-Derivat pro kg Lebendgewicht z.B. von mindestens 0,16 µg Canadin und/oder Canadin-Derivat pro kg Lebendgewicht, z.B. von mindestens 1 µg Canadin und/oder Canadin-Derivat pro kg Lebendgewicht verabreicht wird. Typischerweise überschreitet die Menge des pro Tag verabreichten Canadins und/oder Canadin-Derivat nicht 4,8 µg Canadin und/oder CanadinDerivat pro kg Lebendgewicht. Die vergleichsweise hohe Konzentration von Canad in bzw. Canadin-Derivat von um die 5 µg pro kg Lebendgewicht kann insbesondere für die Behandlung pathologischer physiologischer Zustände bzw. eines stark erhöhten Niveaus an oxidativem Stress verwendet werden. Eine noch höhere Dosierung ist unschädlich. Beispielsweise wurde beobachtet, dass Canadin und/oder Canadin-Derivat zur Behandlung besonders hohem oxidativen Stress in einer Menge von über 5 µg, z.B. von über 1 mg, z.B. auch über 50 mg Canadin und/oder Canadin-Derivat pro Tag und kg Lebendgewicht des Tieres verabreicht werden kann, ohne dass unerwünschte Nebenwirkungen auftraten.

Es wurde beobachtet, dass die besagten geringen Canadin- und/oder Canadin-Derivat-Mengen geeignet sind, das Muskel/Fett-Verhältnis zu verbessern, und das Niveau oxidativen Stresses im Gewebe der Tiere (der relevant ist für die Lagerfähigkeit und Qualität des Fleisches) zu reduzieren.

Falls das besagte Futtermittel, Tränkwasser oder Futterzusatz der Behandlung von pathologischen, also medizinisch behandlungsbedürftigen, Stoffwechselzuständen verwendet wird, z.B. zur Behandlung von pathologischem oxidativen Stress, kann die Konzentration an Canadin und/oder Canadin-Derivat pro kg Lebendgewicht und Tag auch deutlich höher gewählt werden, z.B. so, dass die Konzentration mindestens zweimal oder mindestens fünfmal, oder mindestens 10 mal über den oben genannten Mindestkonzentrationsangaben liegt.

Nach Ausführungsformen enthält das Tierfutter oder Tränkwasser zumindest 0,16 µg Canadin und/oder Canadin-Derivat pro kg Tierfutter bzw. Tränkwasser, insbesondere mit zumindest 1,6 µg Canadin und/oder Canadin-Derivat pro kg Tierfutter bzw. Tränkwasser. Typischerweise enthält das Tierfutter bzw. Tränkwasser 0,1 bis 50 µg Canadin und/oder Canadin-Derivat pro kg Tierfutter bzw. Tränkwasser. Die Konzentration des Canadins in dem Tierfutterzusatzmittel ist vorzugsweise so gewählt, dass das Tierfutterzusatzmittel bei bestimmungsgemäßer Verwendung und Beimengung zu einem Futter oder Tränkwasser ein Tierfutter bzw. Tränkwasser mit den oben genannten Canadin- bzw. und/oder Canadin-Derivat-Konzentrationen ergibt.

Nach Ausführungsformen enthält das Tierfutters oder Tränkwasser zusätzlich Berberrubin.

Gemäß manchen Ausführungsformen handelt es sich um Tierfutter oder Tränkwasser mit zumindest 0,01 µg Berberrubin pro kg Tierfutter bzw. Tränkwasser, insbesondere mit zumindest 0,1 µg Berberrubin pro kg Tierfutter bzw. Tränkwasser.

Nach anderen Ausführungsformen handelt es sich um einen Tierfutterzusatz, dessen Berberrubinkonzentration so gewählt ist, dass bei bestimmungsgemäßer Beimengung zu einem Tierfutter oder zu einem Tränkwasser ein Tierfutter oder Tränkwasser mit zumindest 0,01 µg Berberrubin pro kg Tierfutter bzw. Tränkwasser, insbesondere mit zumindest 0,1 µg Berberrubin pro kg Tierfutter bzw. Tränkwasser, ergibt.

Unter **"Pflanzenmaterial"** wird hier Material verstanden, welches ganz oder teilweise pflanzlichen Ursprungs ist. Beispielsweise kann es sich bei dem Pflanzenmaterial um Teile von Pflanzen, z.B. pulverisierte Blätter oder Stengel, handeln. Es kann sich auch um flüssiges Material handeln, z.B. um einen Pflanzenextrakt, insbesondere einen alkoholbasierten Pflanzenextrakt, z.B. einen ethanolbasierten Pflanzenextrakt

### Kurzbeschreibung der Figuren

Im Folgenden werden Ausführungsformen der Erfindung nur exemplarisch näher erläutert, wobei auf die Zeichnungen verwiesen wird, in denen sie enthalten sind. Es zeigen:
- Figur 1: eine Tabelle mit Ergebnissen eines Fütterungsversuchs mit Schweinen;
- Figur 2: ein Säulendiagramm zur Darstellung des Ergebnisses des Fütterungsversuchs gemäß Figur 1;
- Figur 3: eine Tabelle zur Darstellung von Ergebnissen eines weiteren Fütterungsversuchs mit Masthähnchen;
- Figur 4: ein Säulendiagramm zur Darstellung der Ergebnisse der Tabelle in Figur 3;
- Figur 5: ein Säulendiagramm zur Darstellung des Effekts unterschiedlicher Canadin-Konzentrationen auf den Protein- und Fettgehalt von Hähnchenbrustfleisch;
- Figur 6: ein Säulendiagramm zur Darstellung des Effekts verschiedener Mengen Canadin auf Malondialdehyd (MDA) bzw. oxidativen Stress;
- Figur 7: zeigt den Effekt von Canadin auf die Brust- und Beinmuskulatur der Versuchstiere;
- Figur 8: zeigt die Strukturformel von Canadin; und
- Figur 9: zeigt die Strukturformel von (S)-N-Methylcanadin.

### Ausführliche Beschreibung

Die nachfolgenden Beschreibungen beziehen sich auf verschiedene Versuche, in welchen ein erfindungsgemäßes Futtermittel eingesetzt wurde. Die entsprechenden Vorteile und Wirksamkeiten treffen analog auch auf ein Tränkwasser mit entsprechener Alkaloidmenge bzw. Konzentration zu, sowie auf ein Tierfutterzusatzmittel zur Herstellung des besagten Tierfutters.

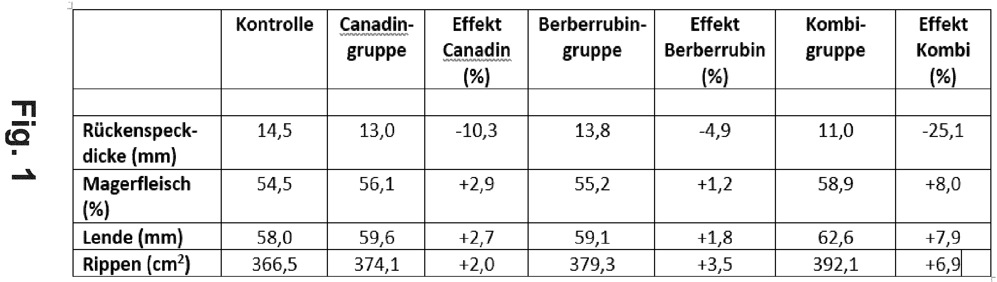

**Figur 1** zeigt eine Tabellen mit Ergebnissen eines Fütterungsversuches.

Es wuren 995 Mastschweine, die männliche wie auch weibliche Tiere umfassten, in vier etwa gleich große Gruppen aufgeteilt. Eine als "Kontrolle" bezeichnete Kontrollgruppe wurde 30 Tage lang bis zum Tag der Schlachtug mit Mastfutter ohne Canadinzusatz (und ohne Zusatz sonstiger Alkaloide) gefüttert (Basaldiät auf Weizenbasis). Die als "Canadin"-gruppe" bezeichnete Gruppe erhielt ab dem 30ten Tag vor dem Schlachttermin ein Mastfutter, das die gleiche Zusammensetzung wie das Futter der Kontrollgruppe hatte, welches aber zusätzlich N-methyl-Canadin in einer Konzentration von 35 µg N-methyl-Canadin pro kg Futter enthielt. Berberrubin war in dem Futter der Canadin-Gruppe nicht enthalten. Die als "Berberrubin"-gruppe" bezeichnete Gruppe erhielt ab dem 30ten Tag vor dem Schlachttermin ein Mastfutter, das die gleiche Zusammensetzung wie das Futter der Kontrollgruppe hatte, welches aber zusätzlich Berberrubin in einer Konzentration von 35 µg Berberrubin pro kg Futter enthielt. Canadin war in dem Futter der Berberrubin-Gruppe nicht enthalten. Die als "Kombi"-gruppe bezeichnete Gruppe erhielt während der 30 Tage vor dem Schlachttermin ein Mastfutter, das die gleiche Zusammensetzung wie das Futter der Kontrollgruppe hatte, welches aber zusätzlich eine Kombination von N-methyl-Canadin und Berberrubin in einer Konzentration von 35 µg N-methyl-Canadin pro kg Futter und von 35 µg Berberrubin pro kg Futter enthielt.

Die Tiere wurden bis zum Schlachttag mit dem jeweiligen Futter der Gruppe ad libitum gefüttert, sie konnten also soviel Nahrung aufnehmen wie sie wollten. Nach der Schlachtung wurden verschiedene Schlachtkörperteile vermessen und die Ergebnisse in der Tabelle festgehalten.

Im Ergebnis führte die Verwendung eines N-methyl-Canadin-haltigen Futters beim Schwein zu einem höheren Schlachtgewicht (110 kg - 112 kg). Die Dicke des Rückenfettes reduzierte sich deutlich (14,5 mm - 13 mm, -10,3 %). Das Verhältnis von Muskel-zu-Fett nahm wegen des gesteigerten Muskelwachstums und wegen der Reduktion des Fettanteils deutlich zu. An der Lende ließ sich ein Muskelzuwachs feststellen (+2,7 %) und der Fleischansatz an den Rippen vergrößerte sich deutlich (+2 %). Die Muskeldicke, gemessen in mm, nahm leicht zu. Insgesamt wurde mehr Muskelmasse (Protein) bei verringertem Fettanteil produziert.

Auch in der Berbberrubin-Gruppe konnten die besagten positiven Effekte beobachtet werden.

Es wurde beobachtet, dass die positiven Effekte von N-methyl-Canadin durch die zusätzliche Verabreichung von Berberrubin in ganz erheblichem Umfang auf synergistische Weise verstärkt werden. Beispielsweise ging die Rückenspeckdicke bei Verfütterung einer Kombination aus N-methyl-Canadin und Berberrubin etwa doppelt so stark zurück (-25,1%) als bei der Verwendung eines Futters, dem lediglich N-methyl-Canadin zugesetzt wurde.

Als Grundlage der Berechnung der Differenz-Prozentwerte diente jeweils die Kontrollgruppe

In dem hier dargestellten Versuch wurden N-methyl-Canadin und Berberrubin in einem Verhältnis von 1:1 zueinander verabreicht. Weitere Versuche haben gezeigt, dass auch andere Mengenverhältnisse eine deutliche Verstärkung der Wirkung von Canadin bzw. N-methyl-Canadin bewirken, z.B. ein Mengenverhältnis von Canadin (bzw. Derivat): Berberrubin von 1:1,2 bis 1,2:1, oder auch von 1:5 bis 5:1.

Die beiden Alkaloide lagen im Futter jeweils in Form von Salzen vor, nämlich als Canadin-Chlorid und Berberrubin-Chlorid.

Der Versuch zeigt, dass die Verfütterung von N-methyl-Canadin an Mastschweine ca. 1 Monat vor dem Schlachttermin die Rückenspeckdichte erheblich reduziert, und zudem den Magerfleischanteil erhöht. Körperbereiche wie Lende oder Rippen, die schon von vorneherein einen hohen Muskelfleischanteil haben, wachsen unter der Einwirkung von N-methyl-Canadin deutlich besser als ohne Canadinzugabe. Ein N-methyl-Canadin-haltiges Tierfutter führt beim Schwein also zu einer Verbesserung, nämlich Erhöhung, des Muskel-zu-Fett-Verhältnisses. Dieser positive Effekt wird durch die zusätzliche Verabreichung von Berberrubin in ganz erheblichem Umfang verstärkt.

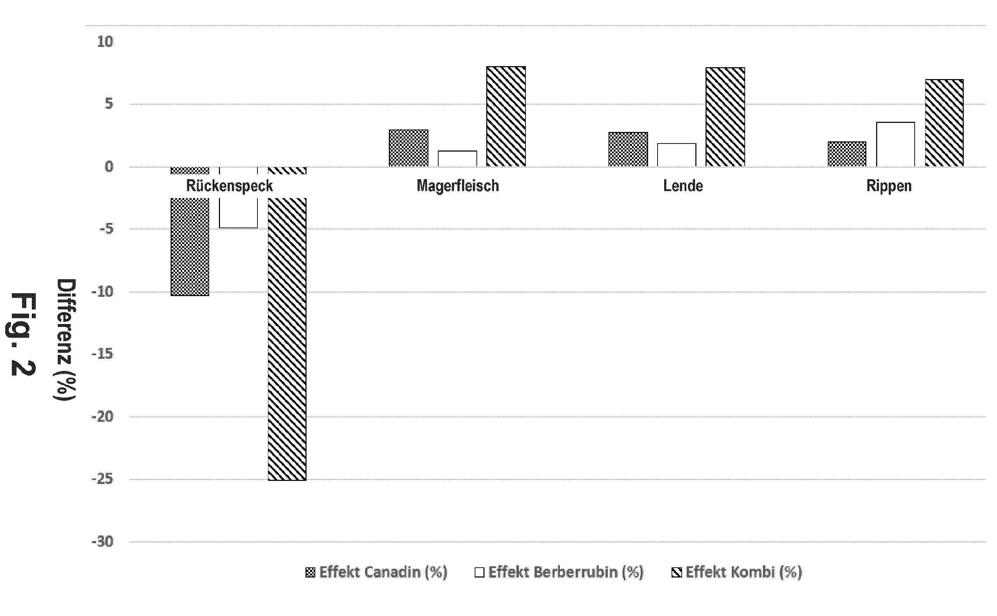

**Figur 2** zeigt die Ergebnisse des im Hinblick auf Figur 1 beschriebenen Fütterungsversuchs in Form eines Säulendiagramms, welches die relativen Änderungen zur Kontrollgruppe ("Differenz") in Prozentwerten wiedergibt. Der synergistische Effekt der kombinierten Verabreichung N-methyl-Canadin und Berberruibin zur Reduktion des Fettgehalts bei gleichzeitiger Erhöhung des Muskelgehalts ist deutlich zu erkennen. Beispielsweise bewirkte N-methyl-Canadin alleine eine Verringerung des Rückenspeckanteils um 10,3 %, Berberrubin alleine um 4,9%, aber eine Kombination beider Substanzen eine Reduktion um über 25%. Ein synergistsicher Effekt war auch im Hinblick auf die Erhöhung des Magerfleischanteils zu beobachten. Aus dem Diagramm ist ebenfalls entnehmbar, N-methyl-Canadin und Berberrubin jeweils alleine bereits entsprechene positive Effekte bewirken.

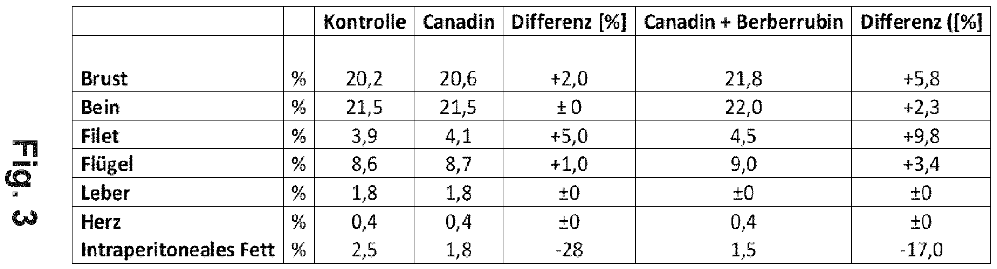

**Figur 3** zeigt eine Tabelle zur Darstellung von Ergebnissen eines weiteren Fütterungsversuchs mit Hühnern. Es wurden 26.400 Cobb 500 Hühner in drei gleich große Gruppen ("Kontrolle", "Canadin" und "Candin+Berberrubin") unterteilt. Alle drei Gruppen wurden über 49 Tage ad libitum mit Futter versorgt. Die Fütterung erfolgte dreiphasig ("starter" Tag 1 bis 10, "grower" Tag 11 - 24, "finisher" 25 - 49) gemäß den während der Entwicklung der Hühner sich verändernden Bedürfnissen an die Futterzusammensetzung. Es wurden Ein-Tages-Küken eingesetzt. Die Diät basiert auf Mais, Sojamehl, Weizen, püriert, und dem jeweiligen Alkaloid-Additiv.

Das Futter der Canadin-Gruppe enthielt zusätzlich 35 µg Canadin pro kg Futter, aber kein Berberrubin. Das Futter der Canadin+Berberrubin-Gruppe enthielt 35 µg Canadin pro kg Futter sowie zusätzlich 35 µg Berberrubin pro kg Futter.

Danach wurden die Hühner geschlachtet und verschiedene Organe bzw. Schlachtkörperteile näher im Hinblick auf den Fett- und Muskelgehalt untersucht.

Die Canadin-Gruppe erzielte insbesondere in den Bereichen Brust, Flügel bzw. Filet zu besseren Schlachtergebnissen, der intraperitoneale Fettgehalt war in der Canadin-Gruppe wie auch in der Canadin+Berberrubin-Gruppe deutlich geringer als in der Kontrollgruppe. So betrug der Fettanteil in der Kontrollgruppe 2,5 %, in der Canadin-Gruppe 1,8 % und in der Canadin+Berberrubin-Gruppe nur 1,5%. Das entspricht einer Differenz des Fettanteils in der Canadin-Gruppe zur Kontrollgruppe von 28 %. Somit war sowohl bei Hühnern wie auch bei Schweinen eine deutliche Verbesserung des Fleisch-zu-Fett Anteils beobachtbar. In der Canadin+Berberrubin-Gruppe wurde eine zusätzliche Verringerung des Fettanteils gegenüber der Canadin-Gruppe von 17% erreicht.

Insgesamt lässt sich wie beim Schwein feststellen, dass Canadin bzw. N-methyl-Canadin zu einem höheren Muskelfleischanteil bei reduziertem Fettgehalt, also einem höheren Magerfleischanteil führt, und dass diese Wirkung durch Berberrubin verstärkt wird.-

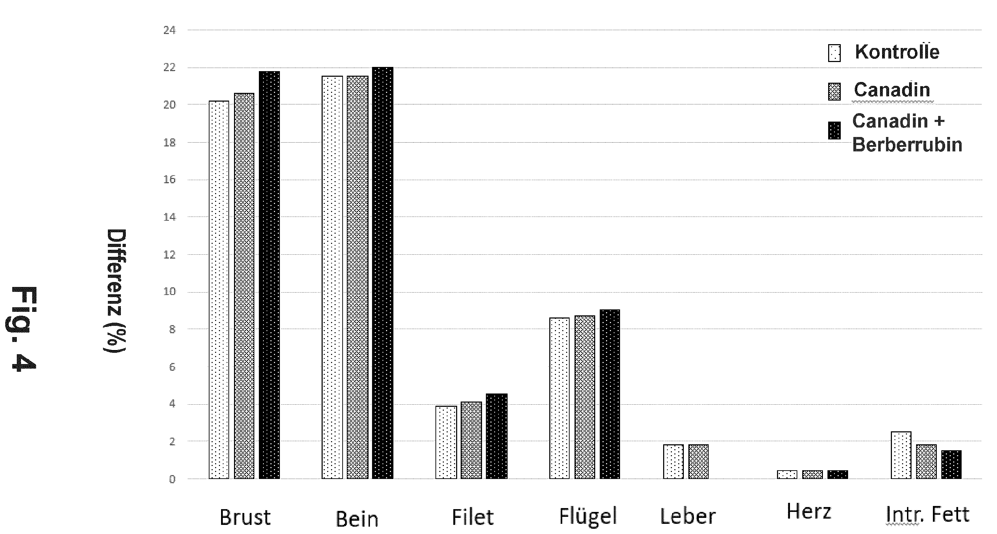

**Figur 4** zeigt ein Säulendiagramm zur Darstellung der in Figur 3 gezeigten Ergebnissen des Versuchs mit den Masthähnchen.

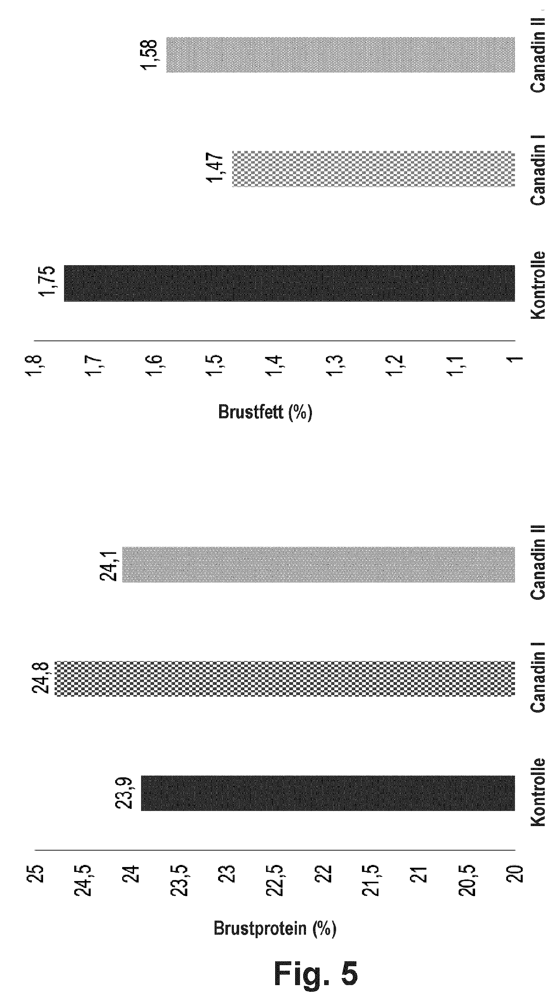

**Figur 5** zeigt die Ergebnisse eines weiteren Versuchs mit Masthähnchen in Form eines Säulendiagramms.

Es wurden 360 Ein-Tages-Küken eingesetzt. Die Diät basiert auf Mais, Sojamehl, Weizen, püriert, und dem Additiv. Der Versuch umfasste drei Wachstums- bzw. Fütterungsphasen: "starter" (Tag 1 bis 10), "grower" (Tag 11 - 24) und "finisher" (Tag 25 - 42); drei Versuchsgruppen - Kontrolle, zwei Konzentrationen, zu je 120 Tieren pro Versuchsansatz. Die Fütterung (ad libitum) erfolgte mit einer Basaldiät, welche je nach Gruppe ergänzt wurde.

Eine als "Kontrolle" bezeichnete Kontrollgruppe wurde mit einem an die jeweilige Wachstumsphase angepassten Basisfutter ohne Canadinzusatz (und ohne Zusatz sonstiger Alkaloide) gefüttert. Das Basisfutter aller Gruppen bestand im Wesentlichen aus Mais, Sojamehl und Weizen. Je nach Gruppe enthielt das Futter ggf. zusätzlich die entsprechende Menge an Alkaloiden. Die als "Canadin I"-gruppe bezeichnete Gruppe bekam ein Basisfutter, das die gleiche Zusammensetzung wie das Futter der Kontrollgruppe hatte, welches aber zusätzlich Canadin in einer Konzentration von 50 µg pro kg Futter enthielt. Berberrubin war in dem Futter der drei Gruppen nicht enthalten. Die als "Canadin II"-gruppe bezeichnete Gruppe erhielt ein Basisfutter, das die gleiche Zusammensetzung wie das Futter der Kontrollgruppe hatte, welches aber Canadin in der Konzentration von 35 µg pro kg Futter enthielt, also weniger als in der "Canadin I" Gruppe.

Die Tiere wurden bis zum Schlachttag mit dem jeweiligen Futter der Gruppe ad libitum gefüttert, sie konnten also soviel Nahrung aufnehmen wie sie wollten. Nach der Schlachtung wurden verschiedene Messungen am Schlachtkörper der Tiere durchgeführt.

So wurde z.B. der Protein- und Fettgehalt vom Brustfilet der Masthähnchen bestimmt. Beim der Canadin-I Gruppe konnte ein gegenüber der Kontrollgruppe erheblich verringerter Fettanteil und ein deutlich erhöhter Muskelanteil nachgewiesen werden.

Beim der Canadin-II Gruppe konnte konnte der gleiche Effekt beobachtet werden, er war jedoch weniger ausgeprägt als in der "Canadin I" Gruppe. Somit konnte gezeigt werden, dass die vorteilhafte Wirkung des Canadins mit dessen Konzentration zunimmt.

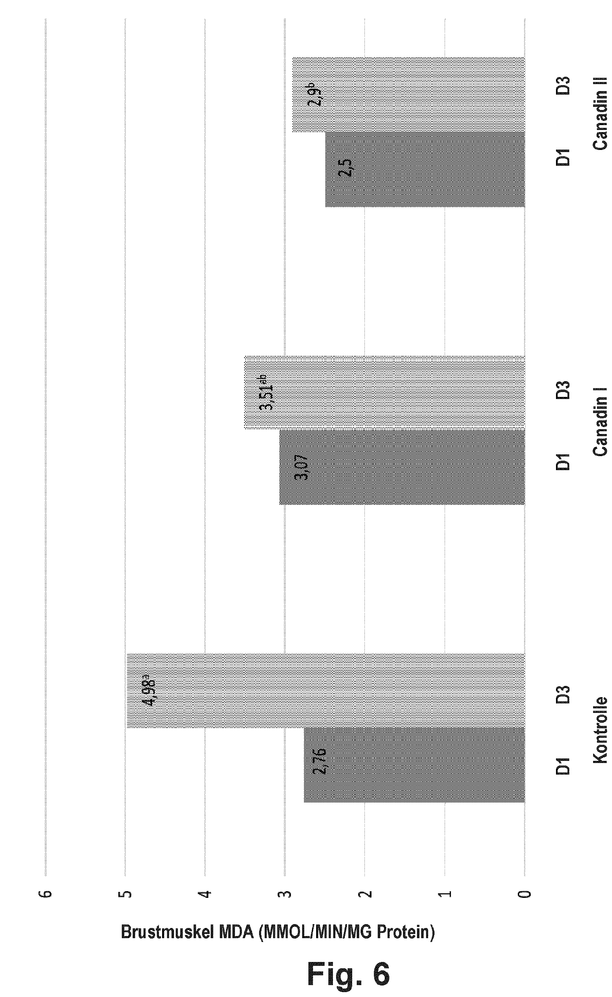

**Figur 6** zeigt, dass Canadin zur Behandlung von oxidativem Stress verwendet werden kann. Dies kann z.B. zu einer Verbesserung der Lagerfähigkeit führen.

Zur Bestimmung des oxidativen Stresses in einem Gewebe wird im Allgemeinen der der Gehalt von Malondialdehyd (MDA) in diesem Gewebe bestimmt. MDA ist also ein Biomarker für die Höhe von oxidativem Stress. Erhöhte MDA Werte im Gewebe können zu Lebzeiten eines Organismus Ausdruck von ungünstigen Lebensbedingungen sein, z.B. falsches Futter, Krankheiten oder ungünstige Haltungsbedingungen. Nach dem Schlachten kann der MDA Wert jedoch auch als Indikator für die Abnahme der Fleischqualität im Zuge der Lagerung dienen. Während der Lagerung steigt nämlich der MDA Gehalt des Gewebes wegen des Eindringens von Luftsauerstoff in das Gewebe und wegen weiterhin im Gewebe ablaufenden biochemischen Reaktionen an. Ein hoher MDA Gehalt gilt daher für ein Indikator, dass das Fleisch schon lange gelagert wurde.

In der Brustmuskulator der Masthähnchen aus dem in Figur 4 beschriebenen Fütterungsversuch wurden nach der Schlachtung der MDA Wert bestimmt. In jeder der drei Gruppen wurde der MDA Wert bei der Hälfte der Tiere nach einem Tag Lagerung im Kühlschrank bestimmt (Untergruppe "D1"), bei der anderen Hälfte der Tiere nach drei Tagen Lagerung im Kühlschank (Untergruppe "D3").

Bei der Kontrollgruppe stieg der MDA Gehalt während der Lagerung im Kühlschrank deutlich an. Dies ist ein Hinweis auf freie Sauerstoffradikale in der Brustmuskulatur der Kontrollgruppe und ein Indiz für eine Abnahme der Lagerfähigkeit bzw. Alterung des Schlachtkörpers.

In den Brustfilets der Masthähnchen der Gruppen "Canadin I" und "Canadin II" fiel dieser Anstieg bis zum dritten Tag ("D3") jedoch erheblich geringer aus. Dies zeigt, dass die Verabreichung von Canadin die Menge der reaktien Sauerstoffverbindungen in der Brustmuskulatur der Masthähnchen und damit die Lagerfähigkeit des Schlachtkörpers erheblich reduzieren konnte. Zu beachten ist, dass die MDA Konzentration bei der Schlachtung bei den verschiedenen Tieren einer gewissen Variabilität unterliegt, sodass für die Lagerfähigkeit insbesondere der relative Zuwachs der MDA Konzentration gemessen am dritten Tag D3 relativ zu dem am ersten Tag der Lagerung (D1) oder zum Schlachttag gemessenen Wert relevant ist. Während der MDA Wert der Kontrolle also sich von D1 nach D3 um über 80% erhöht, nimmt der MDA Wert bei den beiden Canadin-Gruppen nur um etwa 16 % zu.

Eine Reduktion des oxidativen Stresses durch die Verabreichung eines Futters gemäß Ausführungsformen der Erfindung fördert somit nicht nur die Tiergesundheit, sondern führt auch zu einer deutlichen Verbesserung der Lagerfähigkeit.

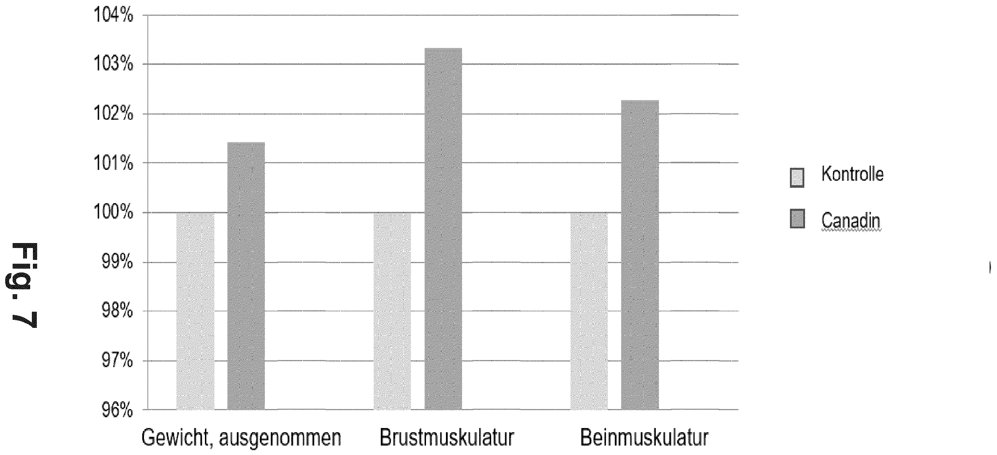

**Figur** 7 zeigt die Ergebnisse eines weiteren Versuchs mit 300 Masthähnchen, die in zwei etwa gleich große Gruppen unterteilt wurden. Die Kontrollgruppe erhielt ein Basisfutter ohne weitere physiologisch aktive Zusätze. Die Canadin-Gruppe erhielt das Basisfutter, welches zusätzlich 35 µg Canadin pro kg Futter enthielt. Die Fütterung wurde gemäß diesem Schema während der Absetzperiode der Masthähnchen (43-49 Tage nach der Geburt) durchgeführt (ad libitum).

Anschließend wurden alle Tiere geschlachtet und verschiedene Parameter bestimmt. Gemessen wurden das Gewicht des Schlachtkörpers, ausgenommen, ohne Innerein, und das Gewicht der Brust- und Beinmuskulatur. Die Schlachtausbeute und das Gewicht der Brustmuskulatur war in der Canadin-Gruppe im Vergleich zur Kontrollgrupe deutlich erhöht.

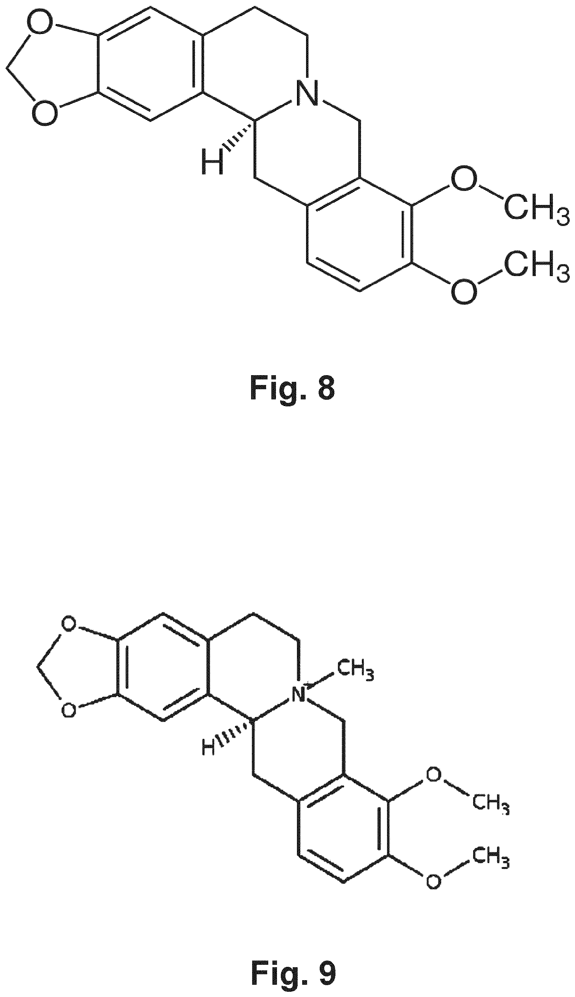

Die **Figuren 8 und 9** zeigen die Strukturformeln von Canadin bzw. von (S)-N-Methyl-canadin. Es wurde festgestellt, dass beide Substanzen die gewünschten Effekte aufweisen, insbeonsdere den Fettanteil im Schlachtkörper reduzieren und den Anteil an Muskelfleisch erhöhen.

Es wurden weitere, ergänzende Versuche WV1, WV*2* und WV3 zur Wirksamkeit von Canadin allein und in Kombination mit Berberrubin durchgeführt. Die in allen drei Versuchen verwendeten Wirkstoffe Methyl-Canadin und Berberrubin wurden durch chromatographische Aufreinigung eines Extrakts des weißen Federmohns gewonnen.

In dem Versuch WV1 wurden 600 Schweine in 7 etwa gleich große Gruppen eingeteilt. Alle Gruppen erhielten während des gesamten nletzten Monats vor dem Schlachttermin das gleiche Basisfutter, die Gruppen unterschieden sich jedoch im Hinblick auf die Menge des verabreichten Canadins (Methyl-Canadin) bzw. Berberrubins.
- Gruppe GK: Kontrollgruppe, erhielt weder Canadin noch Berberrubin
- Gruppe GCA: 5µg Canadin pro kg Futter (kein Berberrubin)
- Gruppe GCB: 10µg Canadin pro kg Futter (kein Berberrubin)
- Gruppe GBA: 20 µg Berberrubin pro kg Futter (kein Canadin)
- Gruppe GBB: 40 µg Berberrubin pro kg Futter (kein Canadin)
- Gruppe GCBA: 5 µg Canadin und 20µg Berberrubin pro kg Futter
- Gruppe GCBB: 10 µg Canadin und 40 µg Berberrubin pro kg Futter

Nach der Schlachtung wurde die Dicke des Rücckenspecks, der Lende, der Rippen und der Magerfleischanteil am gesamten Fleisch bestimmt. Die Ergebnisse des Versuchs sind wie folgt:

| **Gewebe** | **GK Kontrolle** | **GCA Canadin** | **GCB Canadin** | **GBA Berberrubin** | **GBB Berberrubin** | **GCBA Canadin/Berberrubin:** | **GCBB Canadin/Berberrubin** |
|---|---|---|---|---|---|---|---|
| | | **5 µg** | **10 µg** | **20 µg** | **40 µg** | **5 µg/20 µg** | **10 µg/40 µg** |
| **Rückenspeck [mm]** | 14,00 | 12,60 | 12,80 | 12,70 | 12,60 | 11,2 | 11,00 |
| **Magerfleisch [%]** | 53,80 | 55,40 | 56,05 | 55,05 | 55,32 | 57,73 | 58,20 |
| **Lende [mm]** | 56,00 | 57,60 | 57,82 | 57,95 | 58,25 | 59,98 | 59,92 |
| **Rippen [cm2]** | 364,5 | 371,80 | 372,20 | 373,02 | 373,87 | 383,10 | 384,32 |

Dies entspricht einer prozentualen Zunahme bzw. Abnahme relativ zur Kontrollgruppe von:

| **Gewebe** | **GK Kontrolle** | **GCA Canadin** | **GCB Canadin** | **GBA Berberrubin** | **GBB Berberrubin** | **GCBA Canadin/Berberrubin:** | **GCBB Canadin/Berberrubin** |
|---|---|---|---|---|---|---|---|
| | | | | | | **5 µg/20 µg** | **10 µg/40 µg** |
| | **(K)** | **%*** | **%*** | **%*** | **%*** | **%*** | **%*** |
| **Rückenspeck [mm]** | 100 | -10 | -8,57 | -9,28 | -10 | -20 | -21,43 |
| **Magerfleisch [%]** | 100 | +2,9 | +4,18 | +2,3 | +2,82 | +7,30 | +10,82 |
| **Lende [mm]** | 100 | +2,85 | +3,25 | +3,48 | +4,02 | +7,10 | +7,00 |
| **Rippen [cm2]** | 100 | +2,0 | +2,11 | +2,34 | +2,57 | +5,10 | 5,44 |

Auch hier konnte ein synergistischer, über-additiver Effekt der beiden Substanzen beobachtet werden: so wurde z.B. in der Gruppe GCB ein Rückgang des Rückenspecks um -8,57% beobachtet, in der Gruppe GBB um -10%, in der GCBB Gruppe jedoch um -21,43%. Der Magerfleischanteil nahm in den Gruppen GCBA und GCBB deutlich stärker zu, als dies auf Basis der in den Gruppen GCA und GBA bzw. GCB und GBB beobachteten Effekte einzelner Substanzen zu erwarten war.

In einem **weiteren Versuch WV2** wurden 600 Masthänchen in 7 etwa gleich große Gruppen eingeteilt. Alle Gruppen erhielten während der gesamten Start-, Wachstums- und Endphase der Mast das gleiche Basisfutter (pelletieres Alleinfutter), die Gruppen unterschieden sich jedoch im Hinblick auf die Menge des verabreichten Canadins (Methyl-Canadin) bzw. Berberrubins.
- Gruppe GK: Kontrollgruppe, erhielt weder Canadin noch Berberrubin
- Gruppe GCA: 25 µg Canadin pro kg Futter (kein Berberrubin)
- Gruppe GCB: 50 µg Canadin pro kg Futter (kein Berberrubin)
- Gruppe GBA: 50 µg Berberrubin pro kg Futter (kein Canadin)
- Gruppe GBB: 100 µg Berberrubin pro kg Futter (kein Canadin)
- Gruppe GCBA: 25 µg Canadin und \50µg Berberrubin pro kg Futter
- Gruppe GCBB: 50 µg Canadin und 100 µg Berberrubin pro kg Futter

Nach der Schlachtung wurde das Gewicht verschiedener Teile des Schlachtkörpers (als Prozentwert relativ zum Gesamtgewicht) bestimmt. Die Ergebnisse des Versuchs sind wie folgt:

| **Gewebe (jeweils % des Gesamtgewichts)** | **GK Kontrolle** | **GCA Canadin** | **GCB Canadin** | **GBA Berberrubin** | **GBB Berberrubin** | **GCBA Canadin/Berberrubin** | **GCBB Canadin/Berberrubin** |
|---|---|---|---|---|---|---|---|
| | | **25 µg** | **50 µg** | **50 µg** | **100 µg** | **25 µg/50 µg** | **50 µg/100 µg** |
| Brust [%] | 20,7 | 21,3 | 21,5 | 21,6 | 21,8 | 22,3 | 22,4 |
| Bein [%] | 21,1 | 21,2 | 21,6 | 21,9 | 21,7 | 22,4 | 22,5 |
| Filet [%] | 4,0 | 4,2 | 4,3 | 4,5 | 4,6 | 4,7 | 5,0 |
| Flügel [%] | 8,4 | 8,9 | 9,1 | 9,15 | 9,2 | 9,4 | 9,5 |
| Leber [%] | 1,7 | 1,8 | 1,8 | 1,8 | 1,8 | 1,8 | 1,8 |
| Herz [%] | 0,5 | 0,5 | 0,5 | 0,55 | 0,5 | 0,5 | 0,6 |
| Fat (i.p.) [%] | 2,3 | -2,0 | -2,0 | -2,0 | -1,9 | -1,8 | -1,4 |

Werte in Bezug auf die Kontrollgruppe (= 100 %)

| **Gewebe (jeweils % Änderung zur GK)** | **GK Kontrolle** | **GCA Canadin** | **GCB Canadin** | **GBA Berberrubin** | **GBB Berberrubin** | **GCBA Canadin/Berberrubin** | **GCBB Canadin/Berberrubin** |
|---|---|---|---|---|---|---|---|
| | | **25 µg** | **50 µg** | **50 µg** | **100 µg** | **25 µg/50 µg** | **50 µg/100 µg** |
| Brust [%] | 100 | +2,90 | +3,86 | +4,34 | +5,31 | +7,7 | +8,21 |
| Bein [%] | 100 | 10 | +2,37 | +3,79 | +2,84 | +6,16 | +6,64 |
| Filet [%] | 100 | +5,00 | +7,50 | +11,25 | +11,50 | +17,50 | +25,00 |
| Flügel [%] | 100 | +5,96 | +8,33 | +8,93 | +9,52 | +11,90 | +13,10 |
| Leber [%] | 100 | +5,88 | +5,88 | +5,88 | +5,88 | +5,88 | +5,88 |
| Herz [%] | 100 | ±0 | ±0 | ±0 | ±0 | ±0 | ±0 |
| Fat (i.p.) [%] | 100 | -13,04 | -13,04 | -13,10 | -17,39 | -21,74 | -39,13 |

Auch hier konnte ein deutlicher Effekt auf den Muskelanteil (korrespondiert zum Anteil insb. von Brust und Filet) wie auch auf den Fettanteil beobachtet werden. Der Anteil des Brustfleisches und des Files war deutlich erhöht, der Fettgehalt ging bei Verabreichung von Canadin und/oder Berberrubin deutlich zurück. In der GCBB Gruppe sank der Fettgehalt um über 39% relativ zur Kontrollgruppe.

In einem **weiteren Versuch WV3** wurden 500 Masthänchen in 7 etwa gleich große Gruppen eingeteilt und erhielten gruppenspezifisch abgestimmtes Futter gemäß der Versuchsbeschreibung für den Versuch WV2. Nach der Schlachtung wurde das Gewicht verschiedener Teile des Schlachtkörpers (als Prozentwert relativ zum Gesamtgewicht) bestimmt. Die Ergebnisse des Versuchs sind wie folgt:

| | **GK Kontrolle** | **GCA Canadin** | **GCB Canadin** | **GBA Berberrubin** | **GBB Berberrubin** | **GCBA Canadin/Berberrubin** | **GCBB Canadin/Berberrubin** |
|---|---|---|---|---|---|---|---|
| | | **25 µg** | **50 µg** | **50 µg** | **100 µg** | **25 µg/50 µg** | **50 µg/100 µg** |
| Proteinanteil im Brustfleisch [%] | 23,9 | 24,8 | 24,5 | 25,0 | 24,3 | 26,5 | 27,0 |
| Fettanteil im Brustfleisch [%] | 1,75 | 1,66 | 1,62 | 1,65 | 1,63 | 1,60 | 1,58 |
| Abweichung des Proteinanteils im Brustfleisch von GK [%] | 100 | +3,77 | +2,51 | +4,60 | +1,67 | 10,88 | +12,97 |
| Abweichung des Fettanteils im Brustfleisch von GK [%] | 100 | -5,14 | -7,43 | -5,71 | -6,86 | -8,57 | 9,71 |

ES wurde für jede der beiden Substanzen ein deutlich (positiver) Effekt auf den Muskelfleischanteil und ein deutlich (negativer) Effekt auf den Fettanteil beobachtet. Eine Kombination der beiden Substanzen führte insbesondere bei Verwendung höherer Konzentrationen zu einem über-Additiven Effekt auf die Förderung des Muskelanteils bzw. Reduktion des Proteinanteils.

Als besonders vorteilhaft haben sich daher Tränkwasser und Futtermittel erwiesen mit folgenden Beispielwen für Substanzmengen kombinationen (jeweils in µg pro kg Futter bzw. Tränkwasser):

| **Canadin alleine** | **Berberrubin alleine** | **Canadin+Berberrubin kombiniert** |
|---|---|---|
| 5 | 10 | 5+20 |
| 10 | 20 | 25+50 |
| 20 | 40 | 50+100 |
| 25 | 50 | 50+100 |
| 40 | 100 | |
| 50 | | |

Gemäß manchen Beispielen kann das Futter bzw. Tränkwasser also mindestens 5 µg Canadin/kg Futter bzw. Tränkwasser, insbesondere 5-100 µg Canadin/kg Futter bzw. Tränkwasser aufweisen.

Gemäß manchen Beispielen kann das Futter bzw. Tränkwasser also mindestens 10 µg Berberrubin/kg Futter bzw. Tränkwasser, insbesondere 10-150 µg Berberrubin /kg Futter bzw. Tränkwasser aufweisen.

Gemäß manchen Beispielen kann das Futter bzw. Tränkwasser eine Kombination von mindestens 5 µg Canadin und mindestens 10 µg Berberrubin/kg Futter bzw. Tränkwasser aufweisen. Beispielsweise kann das Futter bzw. Tränkwasser eine Kombination von mindestens 1 µg Canadin und mindestens 1 µg Berberrubin /kg Futter bzw. Tränkwasser enthalten. Insebesondere kann das Futter bzw. Tränkwasser eine Kombination von 5-100 µg Canadin und 10-150µg Berberrubin /kg Futter bzw. Tränkwasser enthalten.

Ebenso ist hier ein Futtermittelzusatz offenbart, der bei bestimmungsgemäßer Dosierung eines der hier beschriebenen Tierfutter oder Tränkwässer ergibt.

Ausführungsformen der Erfindung umfassen beispielsweise die folgenden Merkmale:
1. Tierfutter, Tränkwasser oder Tierfutterzusatz beinhaltend Canadin und/oder ein Canadin-Derivat.
2. Tierfutter, Tränkwasser oder Tierfutterzusatz nach Anspruch 1,
   - wobei es sich um Tierfutter oder Tränkwasser mit einer Konzentration von zumindest 0,16 µg Canadin und/oder Canadin-Derivat pro kg Tierfutter bzw. Tränkwasser, insbesondere mit einer Konzentration von zumindest 1,6 µg Canadin und/oder Canadin-Derivat pro kg Tierfutter bzw. Tränkwasser, handelt; oder
   - wobei es sich um einen Tierfutterzusatz handelt, der bei bestimmungsgemäßer Beimengung zu einem Tierfutter oder zu einem Tränkwasser ein Tierfutter bzw. Tränkwasser mit einer Konzentration von zumindest 0,16 µg Canadin und/oder Canadin-Derivat pro kg Tierfutter bzw. Tränkwasser, insbesondere ein Tierfutter bzw. Tränkwasser mit einer Konzentration von zumindest 1,6 µg Canadin und/oder Canadin-Derivat pro kg Tierfutter bzw. Tränkwasser, ergibt.
3. Tierfutter, Tränkwasser oder Tierfutterzusatz nach einem der vorigen Ansprüche, wobei das Tierfutter, der Tierfutterzusatz oder das Tränkwasser auch Berberrubin umfassen.
4. Tierfutter, Tränkwasser oder Tierfutterzusatz nach Anspruch 3, wobei
   - wobei es sich um Tierfutter oder Tränkwasser mit zumindest 1 µg Berberrubin pro kg Tierfutter bzw. Tränkwasser, insbesondere mit zumindest 10 µg Berberrubin pro kg Tierfutter bzw. Tränkwasser, handelt; oder
   - wobei es sich um einen Tierfutterzusatz handelt, der bei bestimmungsgemäßer Beimengung zu einem Tierfutter oder zu einem Tränkwasser ein Tierfutter oder Tränkwasser mit zumindest 1 µg Berberrubin pro kg Tierfutter bzw. Tränkwasser, insbesondere mit zumindest 10 µg Berberrubin pro kg Tierfutter bzw. Tränkwasser, ergibt.
5. Tierfutter oder Tränkwasser nach Anspruch 3 oder 4, wobei dieses eine Kombination von mindestens 1 µg, insbesondere von mindestens 5 µg Canadin und mindestens 1µg, insbesondere mindestens 10 µg Berberrubin/kg Futter bzw. Tränkwasser aufweist, oder Tierfutterzusatz nach Anspruch 3 oder 4, wobei dieses bei bestimmungsgemäßer Dosierung ein Tierfutter oder Tränkwasser mit der besagten Menge an Canadin und Berberrubin ergibt.
6. Tierfutter, Tränkwasser oder Tierfutterzusatz nach einem der vorigen Ansprüche, wobei es sich bei dem Canadin-Derivat um ein zumindest einfach methyliertes Canadin, insbesondere um N-methyl-Canadin, handelt.
7. Tierfutter, Tränkwasser oder Tierfutterzusatz nach einem der vorigen Ansprüche zur Verabreichung an Nutztiere, Haustiere oder Hobbytiere, insbesondere Geflügel, Schweine, Rinder, Schafe, Ziegen, Pferde, Kaninchen.
8. Tierfutter, Tränkwasser oder Tierfutterzusatz nach einem der vorigen Ansprüche,
   - wobei es sich bei Canadin um synthetisches oder biotechnologisch erzeugtes Canadin handelt; und/oder
   - wobei es sich bei Canadin-Derivat um ein synthetisches oder biotechnologisch erzeugtes Canadin-Derivat handelt; und/oder
   - wobei das Canadin als Bestandteil von Pflanzenmaterial in dem Tierfutter, Tränkwasser oder Tierfutterzusatz vorliegt; und/oder
   - wobei das Canadin-Derivat als Bestandteil von Pflanzenmaterial in dem Tierfutter, Tränkwasser oder Tierfutterzusatz vorliegt.
9. Tierfutter, Tränkwasser oder Tierfutterzusatz nach einem der vorigen Ansprüche, wobei das Canadin und/oder das Canadi-Derivat als pysiologisch verträgliches Salz, insbesondere als Chlorid oder Sulfat, vorliegt.
10. Tierfutter, Tränkwasser oder Tierfutterzusatz nach einem der vorigen Ansprüche 1-9 zur Verwendung als Medikament.
11. Tierfutter, Tränkwasser oder Tierfutterzusatz nach einem der vorigen Ansprüche 1-10 zur Reduktion des Fettanteils im Tierkörper.
12. Tierfutter, Tränkwasser oder Tierfutterzusatz nach einem der vorigen Ansprüche 1-10 zur Erhöhung des Muskel-zu-Fett-Verhältnisses im Fleisch von Tieren oder Verwendung eines Tierfutters, Tränkwassers oder Tierfutterzusatzes nach einem der vorigen Ansprüche 1-10 zur Erhöhung des Muskel-zu-Fett-Verhältnisses im Fleisch von Tieren.
13. Tierfutter, Tränkwasser oder Tierfutterzusatz nach einem der vorigen Ansprüche 1-10 zur vorbeugenden oder akuten Behandlung von oxidativem Steress im Gewebe von Tieren, oder Verwendung eines Tierfutters, Tränkwassers oder Tierfutterzusatzes nach einem der vorigen Ansprüche 1-10 zur vorbeugenden oder akuten Behandlung von oxidativem Steress im Gewebe von Tieren.
14. Tierfutter, Tränkwasser oder Tierfutterzusatz nach einem der vorigen Ansprüche 1-10 zur Erhöhung der Lagerfähigkeit des Fleisches geschlachteter Tiere oder Verwendung eines Tierfutters, Tränkwassers oder Tierfutterzusatzes nach einem der vorigen Ansprüche 1-10 zur Erhöhung der Lagerfähigkeit des Fleisches geschlachteter Tiere.
15. Verwendung eines Tierfutters, Tränkwassers oder Tierfutterzusatzes nach einem der vorigen Ansprüche 1-10 zur Reduktion des Fettanteils im Tierkörper.
16. Verwendung eines Tierfutters, Tränkwassers oder Tierfutterzusatzes nach einem der vorigen Ansprüche 1-10 für medizinische Zwecke.
17. Verwendung eines Tierfutterzusatzes nach einem der vorigen Ansprüche 11-16, wobei die Verwendung des Tierfutterzusatzes umfasst eine Beimengung zu einem Tierfutter oder zu einem Tränkwasser in einer solchen Menge, dass ein Tierfutter bzw. Tränkwasser mit einer Konzentration von zumindest 0,16 µg Canadin und/oder Canadin-Derivat pro kg Tierfutter bzw. Tränkwasser, insbesondere von zumindest 1,6 µg Canadin und/oder Canadin-Derivat pro kg Tierfutter bzw. Tränkwasser erzeugt wird.

## Patentansprüche

1. Tierfutter, Tränkwasser oder Tierfutterzusatz beinhaltend Berberrubin und Canadin und/oder ein Canadin-Derivat, wobei es sich bei dem Canadin-Derivat um ein zumindest einfach methyliertes Canadin, insbesondere N-methyl-Canadin, handelt.

2. Tierfutter, Tränkwasser oder Tierfutterzusatz nach Anspruch 1,
- wobei es sich um Tierfutter oder Tränkwasser mit einer Konzentration von zumindest 0,16 µg Canadin und/oder Canadin-Derivat pro kg Tierfutter bzw. Tränkwasser, insbesondere mit einer Konzentration von zumindest 1,6 µg Canadin und/oder Canadin-Derivat pro kg Tierfutter bzw. Tränkwasser, handelt; oder
- wobei es sich um einen Tierfutterzusatz handelt, der bei bestimmungsgemäßer Beimengung zu einem Tierfutter oder zu einem Tränkwasser ein Tierfutter bzw. Tränkwasser mit einer Konzentration von zumindest 0,16 µg Canadin und/oder Canadin-Derivat pro kg Tierfutter bzw. Tränkwasser, insbesondere ein Tierfutter bzw. Tränkwasser mit einer Konzentration von zumindest 1,6 µg Canadin und/oder Canadin-Derivat pro kg Tierfutter bzw. Tränkwasser, ergibt.

3. Tierfutter, Tränkwasser oder Tierfutterzusatz nach einem der vorhergehenden Ansprüche, wobei
- wobei es sich um Tierfutter oder Tränkwasser mit zumindest 1 µg Berberrubin pro kg Tierfutter bzw. Tränkwasser, insbesondere mit zumindest 10 µg Berberrubin pro kg Tierfutter bzw. Tränkwasser, handelt; oder
- wobei es sich um einen Tierfutterzusatz handelt, der bei bestimmungsgemäßer Beimengung zu einem Tierfutter oder zu einem Tränkwasser ein Tierfutter oder Tränkwasser mit zumindest 1 µg Berberrubin pro kg Tierfutter bzw. Tränkwasser, insbesondere mit zumindest 10 µg Berberrubin pro kg Tierfutter bzw. Tränkwasser, ergibt.

4. Tierfutter oder Tränkwasser nach einem der vorigen Ansprüche,
- wobei dieses eine Kombination von mindestens 1 µg, insbesondere von mindestens 5 µg Canadin und mindestens 1µg, insbesondere mindestens 10 µg Berberrubin/kg Futter bzw. Tränkwasser aufweist, wobei das Tierfutter oder Tränkwasser insbesondere eine Kombination von 5-100 µg Canadin und 10-150 µg Berberrubin /kg Futter bzw. Tränkwasser enthalten; oder
- Tierfutterzusatz nach einem der vorigen Ansprüche, wobei dieses bei bestimmungsgemäßer Dosierung ein Tierfutter oder Tränkwasser mit der besagten Menge an Canadin und Berberrubin ergibt.

5. Tierfutter, Tränkwasser oder Tierfutterzusatz nach einem der vorigen Ansprüche zur Verabreichung an Nutztiere, Haustiere oder Hobbytiere, insbesondere Geflügel, Schweine, Rinder, Schafe, Ziegen, Pferde, Kaninchen.

6. Tierfutter, Tränkwasser oder Tierfutterzusatz nach einem der vorigen Ansprüche,
- wobei es sich bei Canadin um synthetisches oder biotechnologisch erzeugtes Canadin handelt; und/oder
- wobei es sich bei Canadin-Derivat um ein synthetisches oder biotechnologisch erzeugtes Canadin-Derivat handelt; und/oder
- wobei das Canadin als Bestandteil von Pflanzenmaterial in dem Tierfutter, Tränkwasser oder Tierfutterzusatz vorliegt; und/oder
- wobei das Canadin-Derivat als Bestandteil von Pflanzenmaterial in dem Tierfutter, Tränkwasser oder Tierfutterzusatz vorliegt.

7. Tierfutter, Tränkwasser oder Tierfutterzusatz nach einem der vorigen Ansprüche, wobei das Canadin und/oder das Canadi-Derivat als pysiologisch verträgliches Salz, insbesondere als Chlorid oder Sulfat, vorliegt.

8. Tierfutter, Tränkwasser oder Tierfutterzusatz nach einem der vorigen Ansprüche 1-7 zur Verwendung als Medikament.

9. Tierfutter, Tränkwasser oder Tierfutterzusatz nach einem der vorigen Ansprüche 1-8 zur Reduktion des Fettanteils im Tierkörper.

10. Tierfutter, Tränkwasser oder Tierfutterzusatz nach einem der vorigen Ansprüche 1-8 zur Erhöhung des Muskel-zu-Fett-Verhältnisses im Fleisch von Tieren oder Verwendung eines Tierfutters, Tränkwassers oder Tierfutterzusatzes nach einem der vorigen Ansprüche 1-8 zur Erhöhung des Muskel-zu-Fett-Verhältnisses im Fleisch von Tieren.

11. Tierfutter, Tränkwasser oder Tierfutterzusatz nach einem der vorigen Ansprüche 1-8 zur vorbeugenden oder akuten Behandlung von oxidativem Steress im Gewebe von Tieren, oder Verwendung eines Tierfutters, Tränkwassers oder Tierfutterzusatzes nach einem der vorigen Ansprüche 1-8 zur vorbeugenden oder akuten Behandlung von oxidativem Steress im Gewebe von Tieren.

12. Tierfutter, Tränkwasser oder Tierfutterzusatz nach einem der vorigen Ansprüche 1-8 zur Erhöhung der Lagerfähigkeit des Fleisches geschlachteter Tiere oder Verwendung eines Tierfutters, Tränkwassers oder Tierfutterzusatzes nach einem der vorigen Ansprüche 1-8 zur Erhöhung der Lagerfähigkeit des Fleisches geschlachteter Tiere.

13. Verwendung eines Tierfutters, Tränkwassers oder Tierfutterzusatzes nach einem der vorigen Ansprüche 1-8 zur Reduktion des Fettanteils im Tierkörper.

14. Verwendung eines Tierfutters, Tränkwassers oder Tierfutterzusatzes nach einem der vorigen Ansprüche 1-8 für medizinische Zwecke.

15. Verwendung eines Tierfutterzusatzes nach einem der vorigen Ansprüche 10-14, wobei die Verwendung des Tierfutterzusatzes umfasst eine Beimengung zu einem Tierfutter oder zu einem Tränkwasser in einer solchen Menge, dass ein Tierfutter bzw. Tränkwasser mit einer Konzentration von zumindest 0,16 µg Canadin und/oder Canadin-Derivat pro kg Tierfutter bzw. Tränkwasser, insbesondere von zumindest 1,6 µg Canadin und/oder Canadin-Derivat pro kg Tierfutter bzw. Tränkwasser erzeugt wird.
